# EUROPEAN PATENT SPECIFICATION

(11) **EP 2 558 436 B1**
(45) Date of publication and mention of the grant of the patent: **07.06.2017**
(21) Application number: 10718689.2
(22) Date of filing: 12.04.2010
(51) Int. Cl.: C07C 69/33, C07D 311/62, A61K 8/37, A61Q 19/00, A61K 31/23

(54) **PROCESS FOR THE MANUFACTURE OF STABILISED POLYPHENOL DERIVATIVES**
VERFAHREN ZUR HERSTELLUNG VON STABILISIERTEN POLYPHENOLDERIVATEN
PROCÉDÉ POUR LA FABRICATION DE DÉRIVÉS DE POLYPHÉNOL STABILISÉS

(43) Date of publication of application: 20.02.2013
(73) Proprietor: Berkem S.A., 24680 Gardonne (FR)
(72) Inventor: PERON, Jean-Louis, F-24680 Lamonzie Saint Martin (FR); NKILIZA, Jean, F-24230 Saint Antoine de Breuilh (FR)
(74) Representative: Thurgood, Alexander John
(86) International application number: PCT/IB2010/000806
(87) International publication number: WO 2011/128714

(56) References cited:
- EP-A1- 0 698 595
- WO-A1-2007/041891
- FR-A1- 2 781 675
- US-A1- 2009 215 881
- HIIPAKKA R A ET AL: "Structure-activity relationships for inhibition of human 5-alpha-reductases by polyphenols", BIOCHEMICAL PHARMACOLOGY, PERGAMON, OXFORD, GB, vol. 63, no. 6, 1 January 2002 (2002-01-01), pages 1165-1176, XP002242332, ISSN: 0006-2952, DOI: DOI:10.1016/S0006-2952(02)00848-1
- DATABASE BEILSTEIN [Online] BEILSTEIN INSTITUTE FOR ORGANIC CHEMISTRY, FRANKFURT-MAIN, DE; 1994, XP002616010, Database accession no. brn 6840754 & PHYTOCHEMISTRY, vol. 35, no. 6, 1994, pages 1559-1566,
- DATABASE BEILSTEIN [Online] BEILSTEIN INSTITUTE FOR ORGANIC CHEMISTRY, FRANKFURT-MAIN, DE; 2009, XP002616011, Database accession no. brn 12151465 & MOLECULES, vol. 14, 2009, pages 4669-4681,

## Description

The present invention relates to processes for the production, of stabilised derivatives of polyphenols.

It is known from the available literature that phenolic compounds are unstable due to the presence of the phenolic functional groups which can be oxidized through various reagents present in the surrounding environment, such as oxygen in air, light, notably ultraviolet light, and certain metallic elements, or they can simply ionize in basic media.

The oxidation process generally involves the creation of free radicals as represented hereafter:

ROH + O₂ → RO^{·} + ^{·}OOH

This mechanism is one of homolytic splitting of the OH bond, giving rise to hydroperoxide (OOH) and alkoxide (RO) free radicals.

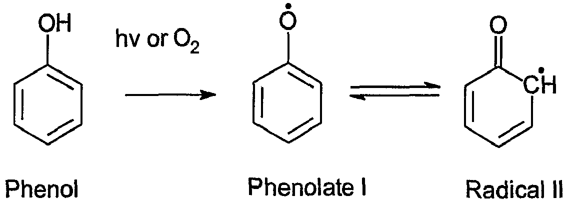

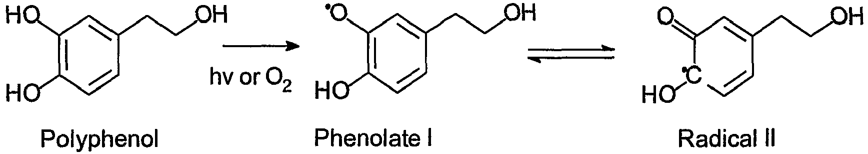

### Example of radical oxidation of polyphenols

The phenolate radical (I) is stabilised through resonance to give radical (II).

Since these radical species are highly reactive, they can couple with themselves in various positions to lead to a variety of products, a few examples of which are given hereafter.

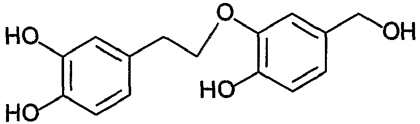

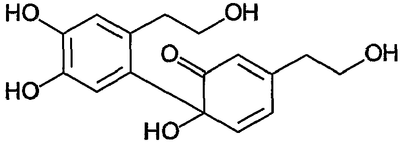

Such a mechanism leads to radical condensation products, and in addition thereto, derivatives of quinones which form when aromatic ring condensation is impossible.

Polyphenol degradation can also occur as a result of change in pH. In basic media, the acidic nature of the phenolic functional groups facilitates exchanges of protons through heterolytic splitting of the OH bond. This leads to the formation of a phenolate anion in equilibrium with the quinone anion.

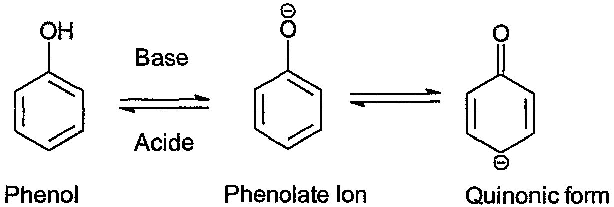

Where proanthocyanidines are present in acidic media, depolymerisation occurs through breakage of the interflavonic bond, i.e. between two monomers, leading to a monomer and a cation where initially there was a dimer. The cation can be oxidized to give anthocyans or will stabilise itself though resonance in the quinone form.

When present in basic media, for example as a catechin monomer, there can be proton exchange, and ring opening of the pyranic ring. Both the phenolate anion and the quinone form will undergo a rearrangement of the backbone to give structures similar to catechinic acid, for example, and/or an epimerisation at the C2 carbon atom, resulting from the conversion of catechin to epicatechin.

Thus it can be seen that the reactivity of polyphenols leads to a range of derivatives, most of which are coloured and of varying colour intensity over time, and due to their instability, are incompatible with usage in certain applications such as cosmetic formulations.

This type of degradation, especially the radical oxidation and basic media reactions, is markedly increased when the aromatic hydroxyl (OH) groups are present as free functional groups because of their strong tendency to exchange hydrogen atoms.

When the phenolic functional groups are protected, for example as esters or ethers, this degradation is to all intents and purposes inhibited :

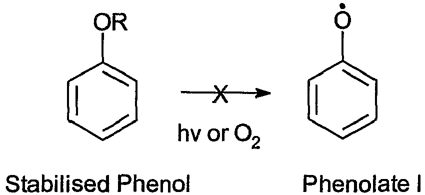

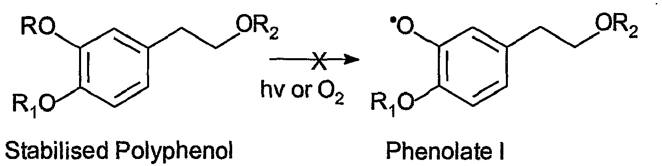

In this schema, R is different to H, for example a linear or branched, saturated or unsaturated, acyl, or alkyl, group.

Another disadvantage of free phenolic functional groups is the increase in the hydrophilic nature of the compound, which is often incompatible with certain excipients used in cosmetic formulations where the lipophilic nature of the formulations is usually predominant. Yet again, esterification or etherification of the free hydroxyl polyphenol functional groups, as is known in the prior art, can be a way around this problem, as can microencapsulation.

Most polyphenols are known to have astringent characteristics. This astringency can lead to a sensation of dryness in the mouth when polyphenols are used in compositions or formulations that are orally ingested or applied to the mucous membranes, and as a result these formulations are not well tolerated or accepted by the consumer. An article by LESSCHAEVE I. & al, published in *Am. J .Clin. Nutr.,* **81**, 330S-335S, 2005, discusses the astringent nature of the majority of polyphenols, and the fact that in the case of proanthocyanidines, this astringent nature is a result of the affinity of the polyphenol for salivary proteins. Interactions between polyphenols and proteins are caused by several factors, of which one of mention are the hydrogen bonds which form between phenolic hydroxyl groups and the nucleophilic sites, i.e. nitrogen and sulphur, of the proteins. This complexation has a negative incidence on the absorptivity and digestibility of macromolecules such as proteins. Indeed, several studies have shown that this property is responsible for the anti-nutritional effects of polyphenols in man and animals, cf. For example, Haslam E., Plant polyphenols, Cambridge university press, 1989 ; MEHANSHO, H. & al, J.Biol.Chem., 260, 4418-4423, 1985; MITJAVILA, S. & al, J.Nutr., 107, 2113-2121, 1977, CHANG M.J. & al, J.Nutr., 124, 283-288, 1994 ; AHMED A.E. & al, Br J.Nutr., 65, 189-197, 1991. Another such anti-nutritional effect is the binding or complexation of metal ions such as iron and copper with polyphenols, leading to reduced availability of these ions for release in the cell.

Several studies have also shown that the catechin polyphenols have remarkable antioxidant properties. According to these studies, a relationship has been discovered between polyphenol structure and anti-radical activity. Phenolic hydroxyl groups are considered responsible for antioxidant activity. Indeed, the apparent precondition is the presence or availability of two free aromatic hydroxyl groups in the ortho position on ring B (see schema below of flavan-3-ol skeleton), which confers increased stability to the phenolate radical through delocalization of electrons, or stabilisation through resonance; but also the presence of a double bond between carbon atom C2 and C3 conjugated with a carbonyl group on carbon atom C4, two hydroxyl groups at positions 3 and 5 of ring A and a carbonyl at carbon atom C4 of ring C. Molecules which fulfil these criteria have been found to be particularly active against radicals. However, the presence of a double bond between carbon atoms C2 and C3, for example as found in rutin, and non-aromatic hydroxyl groups, for example as found on carbon atom 3, would appear to have no significant influence on the molecules overall anti-radical properties.

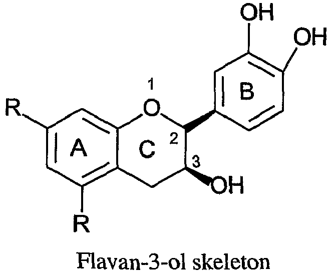

In order to obtain anti-radical activity, it would thus appear necessary to have at least one free phenolic hydroxyl group function. If the polyphenol were completely esterified, it might reasonably be assumed that there would be no residual antioxidant activity due to the fact that proton exchange would be deemed impossible. For this reason, previous prior art solutions have always carried out incomplete protection of the phenolic hydroxyl functional groups, thereby leaving some of these functional groups free.

One such example is described in WO 2007144368 (LIBRAGEN). This patent application describes an enzymatic glycosylation process which enables attachment of a single sugar, i.e. glucose, onto a non-aromatic hydroxyl group, in this case on carbon atom 3. However, glycosylation is known to reinforce the hydrophilic nature of the substrate to which it is attached, and thus the resulting derivative is unsuitable for substantially lipophilic cosmetic or even food preparations. Since the phenolic functions remain free, the derivatives obtained via the process of this patent application can be oxidized like any other phenol, and therefore will suffer from the stability problems over time. The same kind of product is described in US 2007184098 (COGNIS) and EP 1950210 (POLARIS). These patent applications describe esterification processes catalysed by a lipase enzyme and fatty acids. Since the enzymes described are very specific for their substrates, only the non-aromatic hydroxyl groups are esterified. Similar results were also described by PASSICOS E. & al, Biotechnology Letters, 26, 1073-1076, 2004 and TORRES DE PINEDO, A. & al, Tetrahedron, 61, 7654-7660, 2005.

Using these techniques, the phenolic functions remain oxidizable and are also responsible for the solubility of the compounds in polar solvents. However, these compounds remain by the same token poorly lipophilic, and their instability over time renders them incompatible with use in cosmetic applications or lipid based environments.

US 20090215881 discloses cosmetic and therapeutic, in particular dermatological bioprecursors having the formula [A]n-PP-[B]m wherein PP is a polyphenol radical in which each hydroxyl function is protected by a group A or a group B, A is a saturated or unsaturated, substituted or unsubstituted alkyl radical having 1 to 20 carbon atoms which is bonded to the polyphenol, n is an integer not less than 1, and B is a precursor of a biologically active molecule, which is also bonded to the polyphenol, and m is an integer also not less than 1.

EP0698595 discloses a process for the esterification of an oligomeric polyphenolic extract of vegetable origin by an acid chloride. The extract is mixed with a solvent in which it is insoluble to give a suspension; at least one aliphatic tertiary amine of low boiling point is added with a catalytic quantity of at least one organic base, not pyridine; the fatty acid chloride is added to this, and it is mixed at a temperature below 40 °C then concentrated by evaporation. to give the esterified extract.

FR2781675 discloses a bioactive product of silicon and a vegetable bioflavonoid, in which the silicon is present as a biologically-active SiOH group and/or a bond -Si-O- that is hydrolyzable on contact with living tissues to give SiOH group, and a non-hydrolyzable -Si-C- or Si-Si-bond. The preparation of the bioactive product by reaction of a halogenosilane, alkoxysilane, or functionalized alkyl silane with a bioflavonoid in the absence of water, alcohol and light and under inert atmosphere, in the presence of a low boiling point organic base, is also disclosed.

The present invention therefore proposes to resolve the various problems of the prior art by providing a process for the manufacture of such derivatized polyphenols, while maintaining the desirable properties of the original underivatized polyphenols themselves, such as their antioxidant capability, their beneficial action on collagen, their beneficial action on the microcirculatory blood system, on GAGs (glycosaminoglycans), and on fibroblasts, to name but a few.

The polyphenols have phenolic functional groups, the totality of which, i.e. 100%, are protected, either through esterification or etherification, and as determined by an infrared absorption spectrum showing the absence of any free hydroxyl groups.

The polyphenols as defined in the claims are chosen from the following groups:
- hydroxystilbenes, such as monomeric or oligomeric resveratrols, rhapontin, deoxyrhapontin, piceatannol, and the like;
- hydroxycinnamic acids, such as rosmarinic acid, chlorogenic acids, caffeic acids, ferulic acids;
- simple and analogous phenols, such as hydroxytyrosol, oleuropein, verbascoside;
- flavan-3-ols monomers and oligomers, such as catechin, epicatechin, proanthocyanidine oligomers, gallocatechins, epigallocatechins, and the like;
- flavonols, dihydroflavonols, flavanonols, isoflavones ;
- hydroxychalcones and their derivatives, such as aspalathin;
- hydrolysable tannins such as tannic acid, .nobotannin, potentillin, gallnut extract, and the like.

Preferably, the polyphenol is at least one of the following :
- a monomeric and/or oligomeric proanthocyanidine (OPC) found in the group consisting of green tea extract, grape-seed extract, pine bark extract, potentilla extract, and cocoa bean extract;
- a chalcone found in unfermented rooibois tea extract;
- a hydroxystilbene found in grape vine shoot extract.

Even more preferably, the polyphenol is selected from the group consisting of: 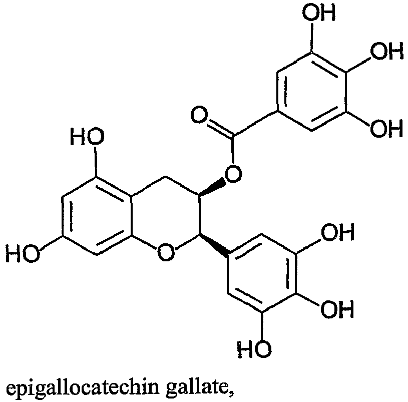 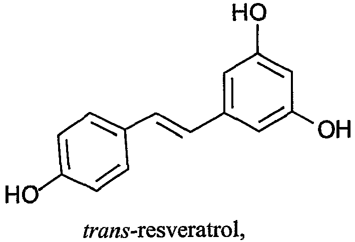 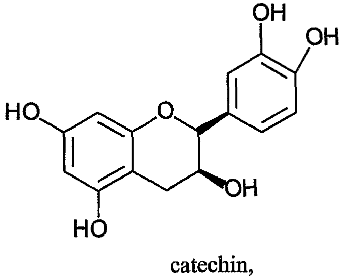 and

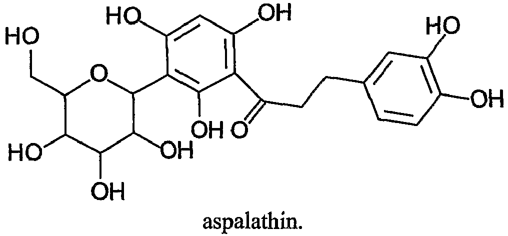

Preferably, the ester group is formed from saturated and unsaturated fatty acid halides containing 6-18 carbon atoms. Exemplary fatty acid halides preferred in this invention are hexanoyl chloride (C6), octanoyl chloride (C8), decanoyl chloride (C10), undecylenoyl chloride (C11), lauroyl chloride (C12), myristoyl chloride (C14), palmitoyl chloride (C16) and oleoyl chloride (C18).

In another preferred embodiment, the ether group is formed from silyl ethers as defined by formulae I and II hereunder: 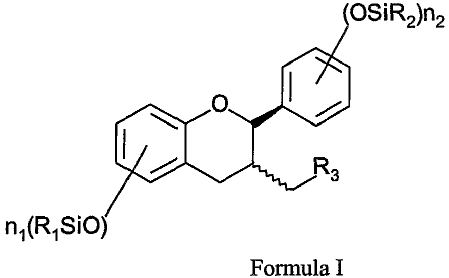 in which
- R₁ and R₂ are identical or different, linked to the Si atom by non-hydrolysable bonds. These radicals being saturated or unsaturated, substituted or unsubstituted hydrocarbons, and when substituted may contain one or more functional groups, such as sterically hindered alkoxy groups. The hydrocarbons can contain from between 1 to 30 carbon atoms;
- R₃ being OH, H, or a silyl ether (OsiR), where R is identical or different to R₁ and R₂ as defined above.
n₁ and n₂ are identical or different, and have a value of from 0 to 3, corresponding to the number of substitutions on a ring ; or 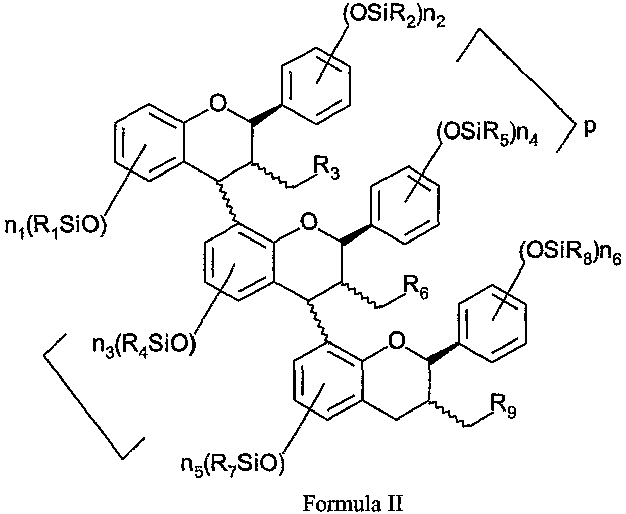 in which:
- n₁, n₂, n₃, n₄, n₅ and n₆ are identical or different, having a value of from 0 to 3 and corresponding to the number of substitutions on a ring, as well as its isomers.
- R₁, R₂, R₄, R₅, R₇, R₈ are identical or different, and linked to the Si atom via non-hydrolysable bonds. They are saturated or unsaturated, substituted or unsubstituted, hydrocarbons. When substituted, they can contain several functional groups, such as sterically hindered alkoxy groups. The hydrocarbons can contain from 1 to 30 carbon atoms ;
- R₃, R₆, and R₉ are identical or different, being OH, H, or a silyl ether (OsiR), where R is identical or different to R₁ and R₂ as defined above;
- p is an integer from 0 to 10.

The compounds obtainable by the process of the present invention have been found to be particularly useful in applications for preventing or treating various nefarious effects caused by free radicals, or glycation of membrane proteins, but are also useful for the protection of the cutaneous extracellular matrix. Said polyphenol derivatives have also been found to reduce or even suppress completely the notoriously astringent characteristics of other polyphenol derivatives known to date, which has previously made them difficult or impossible to use in human or animal food and nutritional applications and formulations.

As mentioned above the present invention relates to a process for the preparation of derivatised polyphenols. It was recently discovered by the applicants that the derivatization conditions used in the the past were not as stable as first thought over time. Usual factors involved in the stability of ester functions are basically linked to the presence of basic or acidic residues in the resulting product, and/or the effect of temperature which causes hydrolysis to occur and thereafter regeneration of the starting reagents. The impurities mentioned here are usually the result of the initial reaction conditions, thereby leading to degradation of the esterification products. In order to ascertain whether this was the case with previous products made by the applicant, grape-seed OPC (oligomeric proanthocyanidine) palmitates made according to a prior process different to the process of the present invention were analysed during storage. The applicant was surprised to find that over time the products analysed displayed a rapid increase in amounts of residual palmitic acid, which in certain cases even exceeded the permitted acceptable norms. The only explanation for this increase was that the ester derivatives were hydrolysing over time because simply put, not all of the phenolic hydroxyl residues had been esterified during the production process and thereby leaving the door open for instability issues.

As the previous production process did not have recourse to any acidic products, this potential reason for the hydrolysis reaction was set aside. The original base used, triethylamine, was traced using gas phase chromatography on several batches of grape-seed OPC palmitates. Non-negligible amounts, up to 2.5%, were found to be present. It was also noticed that the rate of degradation was proportional to the residual quantity of triethylamine in the esterified products. Although several attempts were made to eliminate the triethylamine from the batches, these attempts did not lead to the desired result.

The applicant was thus faced with the problem of devising a new production process that would satisfy several criteria:
- the organic base would have to be easy to eliminate from the reaction mixture without degrading the end product, i.e. the esterified or etherified polyphenol;
- the solvent would have to be considered as non-toxic, which excluded the usual known chlorinated solvents considered as carcinogenic, mutagenic, or having a negative influence on human or animal reproduction.

Accordingly, an object of the present invention is a process for the preparation of derivatized polyphenols comprising causing a polyphenol to react with an ester-forming or ether-forming functional group in the presence of an organic base, wherein :
- the polyphenol is chosen from hydroxystilbenes selected from the group consisting of monomeric or oligomeric resveratrols, rhapontin, deoxyrhapontin, piceatannol; hydroxycinnamic acids, selected from the group consisting of rosmarinic acid, chlorogenic acids, caffeic acids, ferulic acids; simple and analogous phenols, selected from the group consisting of hydroxytyrosol, oleuropein, verbascoside; flavan-3-ols monomers and oligomers, selected from the group consisting of catechin, epicatechm, proanthocyanidine oligomers, gallocatechins, epigallocatechins; flavonols, dihydroflavonols, flavanonols and isoflavones ; hydroxychalcones and their derivatives, preferably aspalathin; and hydrolysable tannins selected from the group consisting of tannic acid, nobotannin, potentillin, gallnut extract, and the like;
- the organic base is selected from the group consisting of imidazole derivatives soluble in water, ethanol and acetone wherein the organic base is chosen from the group consisting of 1-methylimidazole, 2-methylimidazole, 4-methylimidazole, 2-ethylimidazole, 1-ethylimidazole, 1-propylimidazole, and 1-isopropylimidazole and
- the reaction is carried out in an aprotic solvent.

Even more preferably, the polyphenol is selected from the group consisting of : 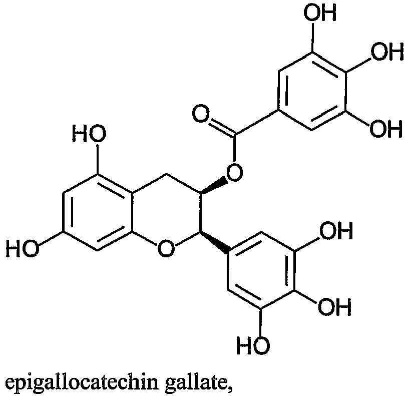 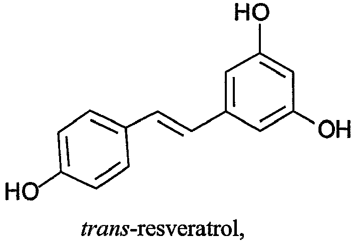 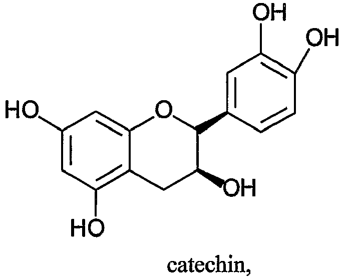 and

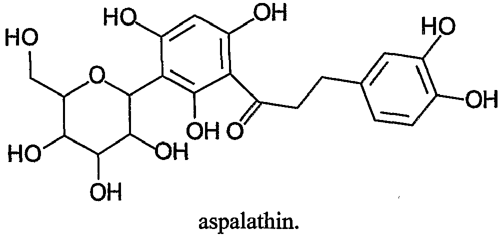

Preferably, the ester-forming group originates from a fatty acid halide selected from the group consisting of saturated and unsaturated fatty acid halides containing 6-18 carbon atoms.

In another preferred embodiment, the ether-forming group originates from silyl ethers as defined by formulae I and II hereunder : 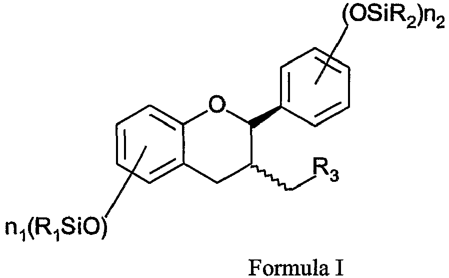 in which
- R₁ and R₂ are identical or different, linked to the Si atom by non-hydrolysable bonds. These radicals being saturated or unsaturated, substituted or unsubstituted hydrocarbons containing from between 1 to 30 carbon atoms, and when substituted may contain one or more functional groups;
- R₃ is OH, H, or a silyl ether (OsiR), where R is identical or different to R₁ and R₂ as defined above.
n₁ and n₂ are identical or different, and have a value of from 0 to 3, corresponding to the number of substitutions on a ring; or 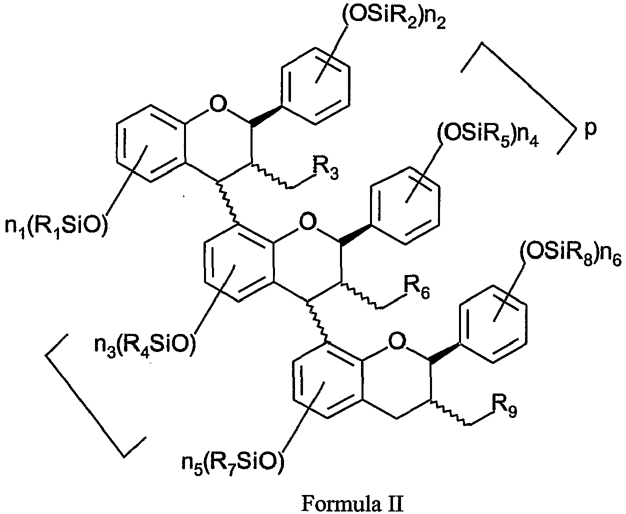 in which:
- n₁, n₂, n₃, n₄, n₅ and n₆ are identical or different, having a value of from 0 to 3 and corresponding to the number of substitutions on a ring, as well as its isomers.
- R₁, R₂, R₄, R₅, R₇, R₈ are identical or different, linked to the Si atom via non-hydrolysable bonds, and are saturated or unsaturated, substituted or unsubstituted, hydrocarbons containing, when substituted, several functional groups, the hydrocarbons containing from 1 to 30 carbon atoms;
- R₃, R₆, and R₉ are identical or different, and can be OH, H, or a silyl ether (OsiR), where R is identical or different to R₁ and R₂ as defined above;
- p is an integer from 0 to 10.

Most preferably, the ether-forming group originates from tertbutyldimethyl chlorosilane.

The preferred solvent used in the process for producing the derivatized polyphenols according to the invention is selected from the group of aprotic solvents consisting of acetone, ethyl acetate, isopropyl acetate, methylethylketone, and isopropyl ether.

The preferred base used in the process for the production of derivatized polyphenols according to the present invention was chosen from imidazole derivatives known to have good solubility in water, ethanol and acetone, and in particular and more preferably chosen from the group consisting of 1-methylimidazole, 2-methylimidazole, 4-methylimidazole, 2-ethylimidazole, 1-ethylimidazole, 1-propylimidazole, and 1-isopropylimidazole.

The present invention will now be described in further detail, referring where applicable and appropriate to the accompanying Figures in which :
- Figure 1 is a representation of the comparison of stability over time between a previous product manufactured by the applicant and the new derivatized polyphenol products obtained via the process of the present invention;
- Figure 2 is an infrared absorption spectrum of a grape-seed polyphenol ester derivative according to the invention;
- Figure 3 is a HPLC trace of esterified grape vine shoot polyphenol;
- Figure 4 is an infrared absorption spectrum of an esterified pine bark polyphenol according to the invention.

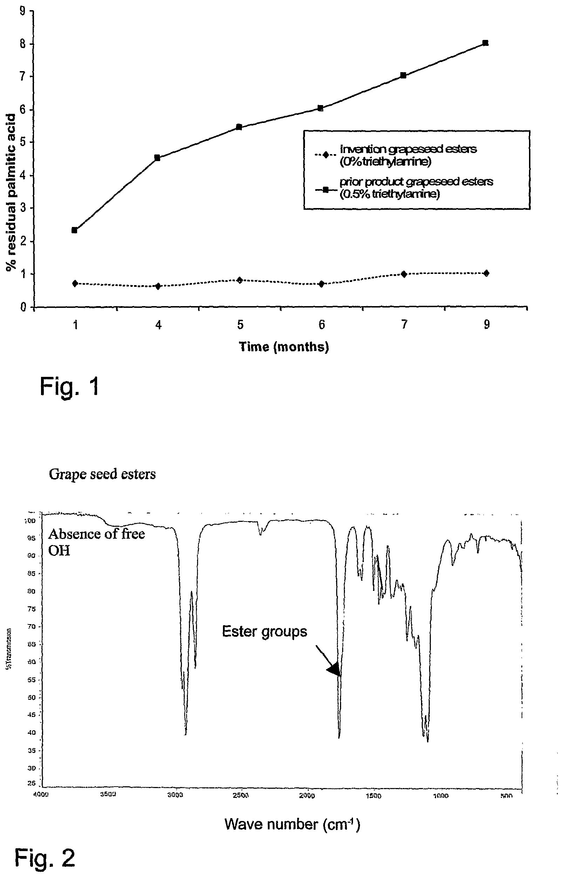

If one looks at Figure 1 identified above, it can be seen that as opposed to the previous products, the new grape-seed polyphenol ester derivatives were found to contain no organic base residues and additionally were totally stable over time, to the extent that no significant evolution in the amounts of residual palmitic acid could be detected. In the following examples, although extracts are used, implying the presence of one or more active phenols, the molar quantities of reactants specified are calculated on the assumption that there is predominantly only one kind of active phenol present in the extract, i.e. catechin for OPCs, epigallocatechin gallate for green tea polyphenols, trans-resveratrol for hydroxystilbens present in grape vine shoot, and aspalathine for the chalcones present in rooibos tea extract.

### Example 1: Preparation of esterified grapeseed OPC.

In a clean and dry three-necked flask with a condenser and a dropping funnel, 10g of grape-seed OPC, representing 34.48 mmole equivalents of catechin, was dissolved in 100 ml acetone, under nitrogen atmosphere. A catalytic amount, 0.6g (5 mmole equivalents), of N,N-dimethylaminopyridine (DMAP) was added and 15 ml (188.41 mmole equivalents) of 1-methylimidazole. The mixture was stirred at room temperature (20-25°C) for about 15 minutes. The dropping funnel was used to slowly add 50 ml (164.23 mmole equivalents) of palmitoyl chloride. The addition lasted about 30 minutes. Stirring was maintained under nitrogen atmosphere and ambient temperature for about 12 hours. The reaction mixture was then concentrated to dryness without exceeding a temperature of 50°C.

The dry residue was taken up in 100 ml 80% ethanol. The mixture was heated to 50°C for about an hour, and then allowed to drop down to ambient temperature (20-25°C). The supernatant was removed and then the remainder washed twice more using the same procedure and conditions.

The washed solid was then dissolved in 100 ml acetone. The warm acetone solution was poured onto 100 ml pure ethanol, and stirred for about 1 hour at ambient temperature (20-25°C), then filtered on number 4 sintered glass having a porosity of 10 to 15 microns, and dried in a vacuum without warming for about 12 hours. A beige powder weighing 30g was obtained. The mass yield of the operation is 300%.

Gaseous phase chromatography analysis showed that 1-methylimidazole was absent and that palmitic acid was only present in a residual amount of 0.5%. The corresponding infrared spectrum, as illustrated in Figure 2, shows no bands above 3000 cm⁻¹ which is the area characteristic of the absorption of free hydroxyl groups, and this indicates that the totality of the functional OH groups are protected by the esterification process. These esters are also shown on the spectrum of Figure 2, at the characteristic bands around 1760 cm⁻¹.

### Example 2: Preparation of esterified grape vine shoots extract

The process described above in example 1 was applied to 4g (17.54 mmole equivalents of *trans-*resveratrol) of polyphenolic extract obtained from grape vine shoots. 13g of stabilised product was obtained as a pale beige coloured powder. The mass yield was approximately 325%.

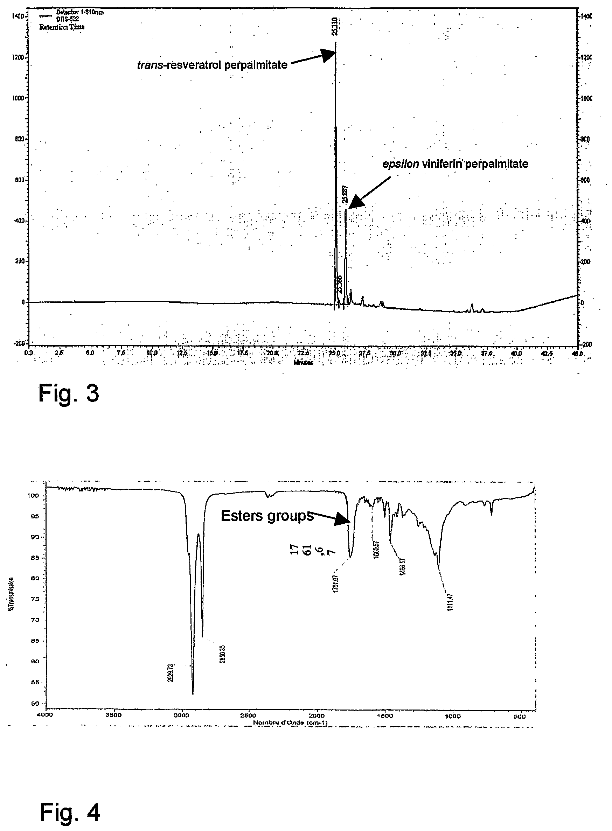

Normal phase HPLC analysis, as illustrated in Figure 3, indicated that the product contains 46.6% of *trans*-resveratrol perpalmitate and 34.6% of *epsilon* viniferin perpalmitate. Gas chromatography indicated a residual palmitic acid content of less than 1% and the complete absence of 1-methylimidazole.

### Example 3 : Preparation of Rooibos tea extract (Aspalathus linears)

500g of non fermented leaves and 2.5 litres of ethanol 50% w/v in distilled water were added to a reaction flask surrounded by a water cooler and equipped with a mechanical stirrer. The mixture was reflux heated for an hour with stirring, and then cooled to ambient temperature (about 20-25°C). Solid-liquid separation was carried out via filtration. A second extraction was carried out under the same conditions.

The two hydroethanolic filtrates were pooled and bleached with activated charcoal to remove chlorophyll. The clear filtrate was concentrated under reduced pressure without exceeding 50°C until all of the ethanol had been removed. The aqueous concentrate, which could still contain trace amounts of matter in suspension was dried directly by atomisation or spray drying. About 75g of dry material was obtained as a beige to orange brown powder. The yield of extraction was approximately 15%. The obtained product absorbs UV light with a maximum absorption at about 280 nm. Total polyphenol content was 32%.

This extract was used to produce an esterified derivative as described in the following example.

### Example 4: Preparation of esterified Rooibos tea extract.

The process described in example 1 was applied to 15g (34.48 mmole equivalents of aspalathin) of the extract prepared as above in example 4. 31 g of beige powder with a greasy consistency was obtained. The mass yield was approximately 200%. The esterified rooibos tea extract derivatives are soluble in apolar solvents such as hexane and absorb UV light with a maximum absorption at 270 nm.

### Exemple 5: Preparation of esterified pine bark OPC.

The process described in example 1 was applied to 10g (34.48 mmole equivalents of catechin) of pine bark OPC, which had been obtained according to the teachings of FR 2 092 743. 25g of a beige powder with a greasy consistency was obtained. The mass yield was approximately 250%. The esterified pine bark OPC was soluble in apolar solvents such as hexane, and absorbs UV light with a maximum absorption at 270 nm.

Gas phase chromatography showed that 1-methylimidazole was absent and that the residual amount of palmitic was 0.4%. The infrared spectrum of Figure 4 showed no bands above 3000 cm⁻¹ where free hydroxyl groups are characteristically located. This indicated that the totality, i.e. 100% of the OH groups were protected by esterification. One of the characteristic bands of the esters is shown on the spectrum at roughly 1760 cm⁻¹

### Example 6: Preparation of esterified potentilla OPC

The process described in example 1 was applied to 15g (51.72 mmole equivalents of catechin) of *P. tormentilla* rhizome OPC extract obtained according to the teachings of FR2749303 or US5928646. About 49g of stabilised product was obtained as a clear beige coloured powder. The mass yield was approximately 325%.

Analysis by gas chromatography showed that 1-methylimidazole was completely absent and that the derivative contained a residual amount of palmitic acid of 2.5%. The IR spectrum showed no bands beyond 3000 cm⁻¹ which is the characteristic location of free hydroxyl groups. All of the OH groups were thus protected via the esterification process, with a characteristic ester band at 1760 cm⁻¹.

### Example 7: Preparation of esterified green tea polyphenols

The process of example 1 was applied to 10 g (21.83 mmole equivalents of epigallocatechin gallate) polyphenolic extract of green tea leaves obtained according to the teaching of FR2734478. The organic base used was 1-ethylimidazole. About 35g of stabilized product was obtained as pale beige powder. The mass yield was about 350%. Gas chromatography showed that 1-ethylimidazole was absent and that there was a residual amount of palmitic acid of 2%. The IR spectrum showed no bands above 3000 cm⁻¹ which is the characteristic location of free hydroxyl groups. All of the OH groups were thus protected by the esterification process.

### Examples 8-13: preparation of esterified grapeseed OPC with derivatives of medium chain fatty acids (C6 to C14).

The following derivatives were used to stabilize the polyphenolic extracts : hexanoyl chloride (C6), octanoyl chloride (C8), decanoyl chloride (C10), undecylenoyl chloride (C11), lauroyl chloride (C12), myristoyl chloride (C14).

The experimental conditions were those used in example 1, with the polyphenolic extract being grape-seed OPC, and reacted with one of the above acid chlorides as acylating agent.

### Example 8 : Grapeseed OPC perhexanoate.

Starting with 10g (34.48 mmole equivalents of catechin) of grapeseed OPC, 18g of esterified product was obtained as a thick brown liquid. Gas chromatograpy analysis showed that 1-methylimidazole was absent, and that only 0.1% of hexanoic acid was present. The IR spectrum showed no bands beyond 3000 cm⁻¹ which is the characteristic location of free OH groups and the presence of bands at 1760 cm⁻¹ indicative of esters.

### Example 9 : Grapeseed OPC peroctanoate.

Starting from 10g (34.48 mmole equivalents of catechin) of grapeseed OPC, it was possible to obtain 23g of esterified product as a thick brown liquid. Gas chromatograpy analysis showed that 1-methylimidazole was absent, and that only 0.4% of octanoic acid was present. The IR spectrum showed no bands beyond 3000 cm⁻¹ which is the characteristic location of free OH groups and the presence of bands at 1760 cm⁻¹ indicative of esters.

### Exemple 10 : Grape-seed OPC perdecanoate.

With 10g (34.48 mmole equivalents of catechin) of starting material, it was possible to obtain 25g of esterified product as a thick brown liquid. Gas chromatography analysis showed that 1-methylimidazole was absent, and that only 0.3% of decanoic acid was present. The IR spectrum showed no bands beyond 3000 cm⁻¹ which is the characteristic location of free OH groups and the presence of bands at 1760 cm⁻¹ indicative of esters.

### Example 11 : Grape-seed OPC perundecylenate.

With 10g (34.48 mmole equivalents of catechin) of starting material, it was possible to obtain 25g of esterified product as a pasty brown solid. Gas chromatography analysis showed that 1-methylimidazole was absent, and that only 1% of undecylenic acid was present. The IR spectrum showed no bands beyond 3000 cm⁻¹ which is the characteristic location of free OH groups and the presence of bands at 1760 cm⁻¹ indicative of esters.

### Example 12 : Grape-seed OPC perlaurate.

With 10g (34.48 mmole equivalents of catechin) of starting material, it was possible to obtain 28g of esterified product as pasty brown solid. Gas chromatograpy analysis showed that 1-methylimidazole was absent, and that only 0.5% of lauric acid was present. The IR spectrum showed no bands beyond 3000 cm⁻¹ which is the characteristic location of free OH groups and the presence of bands at 1760 cm⁻¹ indicative of esters.

### Example 13 : Grapeseed OPC permyristate.

With 10g (34.48 mmole equivalents of catechin) of starting material, it was possible to obtain 23g of esterified product as a pasty brown solid. Gas chromatograpy analysis showed that 1-methylimidazole was absent, and that only 0.5% of myristic acid was present. The IR spectrum showed no bands beyond 3000 cm⁻¹ which is the characteristic location of free OH groups and the presence of bands at 1760 cm⁻¹ indicative of esters.

### Example 14 : Grapeseed OPC peroleate.

With 10g (34.48 mmole equivalents of catechin) of starting material, and under the same general conditions as example 1, with 2-ethylimidazole as organic base and oleoyl chloride as acylating agent, it was possible to obtain 36g of esterified product as an oily light brown liquid. Gas chromatography showed that 2-ethylimidazole was absent, and that 3.5% oleic acid residue remained. The IR spectrum showed no bands beyond 3000 cm⁻¹ which is the characteristic location of free OH groups and the presence of bands at 1760 cm⁻¹ indicative of esters.

### Exemple 15 Preparation of esterified cocoa OPC

The process described in example 1 was applied to 10g (34.48 mmole equivalents of catechin) of cocoa OPC obtained according to the teaching of FR 2 092 743. About 33g of stabilized product was obtained as a pale beige powder. The mass yield was about 330%. Gas chromatography showed the absence of 1-methylimidazole and a residual amount of palmitic acid of 0,3%. The IR spectrum showed no bands beyond 3000 cm⁻¹ which is the characteristic location of free OH groups and presence of bands at 1760 cm⁻¹ indicative of esters.

Example 16 Demonstration of the anti-lipoperoxidant activity of the polyphenol derivatives obtained according to the process of the invention in human skin.

The lipophilic nature of the stabilised polyphenols obtained according to the process of the present invention is relatively high in comparison to the native polyphenols. Added to that is their lack of affinity for proteins, thereby making it possible to consider their topical application directly on the skin for transcutaneous absorption. In this way, the stabilised products come into direct contact with the esterases present in the skin. Much as with lipids, a hydrolytic reaction will occur which will regenerate the polyphenol and a fatty acid residue. The advantageous biological properties of the thus freed polyphenol can then come to the fore, for example as an antioxidant, antiinflammatory, antimicrobial, antiglycation agent, vascular protector, hypocholesterolemia modulator, anti-mitotic agent, etc...

In order to demonstrate this phenomena, the anti-radical activity of the stabilized derivatized polyphenols obtained according to the process of the invention was studied on explants of human skin taken from the abdominal area of a woman aged 40.

Oxygenated free radicals are produced in large quantities on the skin as a result of UV irradiation. These free radical species cause various forms of degradation to cellular components, and in particular, to membrane lipids. The latter are transformed into various hydroperoxide derivatives, such as for example malonyldialdehyde (MDA), and 4-hydrononenal (4-HNE). The determination of the amount of MDA in skin tissues is thus a good indicator of the production of oxygenated free radicals and peroxidation of membrane lipids. A comparison of the levels of MDA between treated and non-treated subjects therefore indicates whether the product applied has anti-lipoperoxidant activity.

### Experimental Conditions:

The model used for the experiments is one of human skin explants which are maintained in survival mode at 37°C, a humid atmosphere and 5% CO₂.

The skin explants are divided into three
- untreated control batch
- positive control batch treated with vitamin E (2 mg per explant)
- batch treated with products according to the invention.

The products to be tested are dissolved at a concentration of 2% Vaseline oil. This solution is applied topically to the skin explants at a rate of one 30 micro-litre application per explant per day for 5 days.

On day 5 the explants are irradiated with UV A and UV B light 2 hours after application of the products to be tested.

Levels of MDA (expressed in pmoles of MDA/ml of medium) in the explants are measured for both the treated and untreated batches.

The table below shows the percent reduction in MDA of treated explants compared to untreated explants.

| **Product Name** | **% Reduction in MDA** |
|---|---|
| Rooibos tea extract palmitic ester | 13 |
| Grape-seed OPC undecylenate ester | 23 |
| Grape-seed OPC palmitic ester | 24 |
| Pine bark OPC palmitic ester | 26 |
| Potentilla OPC palmitic ester | 38 |
| Green tea extract palmitic ester | 45 |
| Vitamin E | 11 |

This study shows that the stabilized polyphenols obtained according to the process of the invention display significant anti-lipoperoxidant activity. All of the esters perform better than the control with vitamin E, which is a known and respected antioxidant reference in cosmetic applications.

Since the anti-radical activity is linked to the presence of free phenolic hydroxyl groups, the results also show that stabilized polyphenols obtained according to the process of the present invention have passed the cutaneous barrier and thereafter been hydrolysed by esterases, thereby freeing the phenolic hydroxyl groups that were initially protected as esters. Another interesting consequence of the above is that the ester derivatives can therefore also be used as vectors for the polyphenols, and for increasing their bioavailability within the body.

### Examples 17 to 20 Demonstration of the protection of extracellular cutaneous matrix components.

In skin, the extracellular matrix is made up of macromolecules which are protein-like or glycan-like in their nature. Generally, 4 main categories of macromolecules can be considered: the collagens, elastin, the proteoglycans (otherwise known as glycosaminoglycans) and the structural glycoproteins (laminins, fibrillins, fibronectin, etc).

The role of collagen is to provide mechanical resistance to cutaneous tissues, elastine is responsible for their elasticity, the glycosaminoglycans deal with hydration, and the structural glycoproteins are responsible for the establishment and cohesion of tissue.

As one gets older, the speed at which most of these macromolecular elements of the extracellular matrix are replaced diminishes sharply. Thus skin loses its firmness, and its elasticity and unpleasant manifestations thereof appear, such as wrinkles. By stimulating the synthesis of these macromolecules of the cutaneous extracellular matrix, it becomes possible to maintain the skin in its normal state.

The products obtained according to the process of the present invention have therefore been tested ex vivo to see whether they might have any activity in the extracellular matrix. The tests were carried out on explants of human skin that was maintained in a viable state.

### Explant Preparation

Explants of approximate diameter of 10 mm were preapred from an abdominal sample taken from a woman aged 46. The explants were maintained alive at 37°C in humid atmosphere, enriched with 5 % de CO₂.

### Product Application

For each derivatized product to be tested, 3 concentrations were prepared in vaseline oil at concentrations of 0.5, 0.25 and 0,1% respectively. The products to be tested were applied topically at a dosage of 30 µL per explant, on a disk of filter paper, at days 0, 2, 3 and 6. The control explants received no treatment at all.

### Sampling

At day 0, the 3 explants from batch T0 were sampled and prepared in buffered formol.

A day 8, the explants from each batch were sampled and handled in the same way. Immunostaining and specific coloring of the explants was then carried out. Activity was observed morphologically through the optical microscope and using image analysis.

### Example 17 : Action of potentilla OPC esters and pine bark OPC esters on collagen I

The potentilla OPC ester, at a concentration of 0.5% induced a 12% increase in collagen I in the papillary dermis.

Pine bark OPC ester, at a concentration of 0.5%, induced an increase of 20% of collagen I in the papillary dermis.

### Example 18 : action of potentilla OPC esters and pine bark OPC esters on collagen III

Potentilla OPC ester at concentrations of 0.5 and 0.1% respectively induced a respective increase of 33% and 38% in collagen III in the papillary dermis.

Pine bark OPC ester at a concentration of 0.5 and 0.1% respectively induced a respective increase in collagen III in the papillary dermis of 21 and 30%.

### Example 19: Action of esterified potentilla OPC and esterified green tea polyphenols on collagen IV

Esterified green tea extract at concentrations of 0.25 and 0.1% respectively induced an increase of 40% and 18% in collagen IV at the epidermal-dermal junction.

Esterified potentilla OPC at a concentration of 0.1% induced an increase of 26% in collagen IV at the epidermal-dermal junction.

### Example 20: Action of esterified green tea polyphenols on fibrillin-1

Esterified green tea polyphenols at concentrations of 0.5 and 0.25% induced a respective increase of 21 and 13% in fibrillin-1 at the epidermal-dermal junction.

Ageing is a complex process well documented in the literature, and linked to several factors, of which, in addition to genetic factors, the following can be mentioned: environmental factors such as oxygenated free radicals, drops in levels of certain hormones, pollution, tobacco and alcohol consumption, UV light from the sun, etc. Sunlight, for example, stimulates the production of matrix metalloproteinases, also known as MMP. These are enzymes which break down the extracellular matrix of conjunctive tissues, especially in the skin. There are several types known, one of which is interstitial collagenase, also known as MMP1, which breaks down collagen. Others which can be mentioned are stromelysin, or MMP3, which breaks down elastin, gelatinase, or MMP2, which mainly breaks down type I collagen. It is thus known that exposure to sunlight causes a change in the make up of collagen and elastin fibres, leading to a loss of tonus and elasticity in the skin which causes wrinkling. Other nefarious effects of the UV component of sunlight are also known, such as a drop in immunity, changes in melanogenesis, the production of oxygenated free radicals, and some forms of skin cancer. The use of polyphenols to assist in combating the effects of exposure to the sun have also been described because polyphenols can act as an antiradical agent by filtering the absorbed radiation, wherein the polyphenols capture or inhibit initiation or propagation of free radicals, but they also play a role in enzymatic inhibition, and as has been shown above, in stimulating the synthesis of collagen and elastin.

Oxygenated free radicals are highly reactive species, which are present throughout the lives of animals and humans, and they also participate in the normal function of the organism, as for example in the respiratory system. They are formed continuously in a healthy organism, most notably within mitochondria. This production is generally balanced out by their uptake by endogenous antioxidants, by enzymes such as superoxyde dismutase (SOD), catalase, and glutathione peroxidase. However, under certain conditions, the balance is disturbed and a situation known as oxidative stress is created. Such stress can be caused by many agents, including UV light, air pollutants such as NO and NO2, organic solvents, pesticides, hyperoxemia, etc. This oxidative stress is known to be involved in a number of pathologies, among which one can mention neurodegenerative pathologies such as Alzheimer's, Parkinson's disease, cardivascular illnesses such as ischemia, mitotic disorders leading to the appearance of tumours and other malformations, cellular ageing, oxidative breakdown of macromolecules, accumulation of inter and toxic intermediates resulting from oxidation of unsaturated lipids and peroxidation of membrane lipids, etc.

The mechanism by which these radicals, usually resulting in a chain reaction, are formed, is as follows:
Initiation (I):

   RH ⇒ R^{·}

The initiation step can be catalysed by several factors, for example UV light.

Propagation (II):

R^{·}+O2 ⇒ ROO^{·}

ROO^{·}+RH ⇒ ROOH + R^{·}

Termination (III):

ROO^{·}+ROO^{·} ⇒ Inert product (dismutation)

R^{·}+ R^{·} ⇒ Inert product (dismutation)

ROO^{·}+R^{·} ⇒ Inert product

In aerobic organisms such as animals and humans, free radicals are essentially oxygenated radicals leading to peroxidation of cell components such as the sugars, lipids, proteins, and DNA, and giving rise to highly unstable products. The radical formation process is self-maintained until a stable end product is attained as indicated above or when a free radical sponge captures the radical at the initiation or propagation stage.

The results of the present invention show that the derivatized polyphenols of the present invention retain all of the beneficial properties associated with the original, unmodified polyphenols, and thus can be used for example in cosmetic formulations intended to prevent or repair the effects of cutaneous ageing.

Other causes of cellular ageing, particularly in relation to the skin can be found in what is known as advanced glycation end products. These products are the result of spontaneous reaction, i.e. without the requirement for enzymes, between glucose and molecules such as collagen and elastin. These end products are more resistant to proteolysis and thus slow down renewal of the components of the extracellular matrix. Additionally, these end products induce reticulation between collagen fibres, making them less soluble and disturbing their organisation within the matrix. The overall imbalance caused leads to loss of tonus and elasticity in the dermis, and is one of the factors responsible for the appearance of wrinkles. As the derivatized polyphenols of the present invention cross the transcutaneous barrier, and the active form is recreated by the action of esterases, they can be used to treat the effects of ageing caused by the glycation reactions mentioned above.

### Example 21 : Demonstration of anti-lipoperoxidant activity of etherified polyphenols obtained according to the process of the invention in human skin

Antiradical activity was determined using the same method described in example 17. An etherified (silyl ether) OPC similar to that described in FR2781675, and corresponding to the formulae I and II below was used :
The results obtained with this derivatized product according to the invention were as follows :

| Product name | % Reduction in MDA |
|---|---|
| Silyletherified grape-seed OPC | 11 |
| Vitamine E | 11 |

As can be seen from the above results, the activity of the product obtained according to the process of the invention was the same as for vitamin E, the standard reference in this field. This also shows that the etherified bonds can be hydrolysed to regenerate the required polyphenol and additionally, silanol. This study also demonstrates that the polyphenol silyl ethers obtained according to the process of the present invention, notably the flavan-3-ol monomers or oligomers can be used in cosmetic formulations, with the additional advantage of combining the benefits of polyphenols with silicon. During hydrolysis, the silicon atom forms a silanol, which is known to be one of the biologically active forms of silicon in vivo.

### Exemple 22 : Cytotoxic analysis of derivatized polyphenols

This test involves determining the viability of cells through colorimetric reaction with tetrazolium salt (MTT). The tetrazolium group, initially showing a yellow colour is reduced to form a formazan group showing a violet blue colour, via the action of mitochondrial succinate dehydrogenase present in active living cells. The medium colour thus changes from yellow to violet blue. The colour intensity measured by optical density at 540 nm is proportional to the number of living and metabolically active cells. This study was carried out on skin explants obtained from abdominal biopsies. The explants are cut into 8mm diameter fragments and maintained alive at 37°C. The products to be studied are dissolved in vaseline oil and the solution applied topically at a dose of 5mg of solution per square centimetre of explant. The concentrations used range from 0.02 to 5%. The trials are carried out in triplicate and contact time of the product with the explants is 24 hours.

The explants are split into 3 groups :
- untreated control group
- control group treated with vaseline oil only
- group treated with product of the invention

Some of the results obtained are given below :

| Treatment | Optical | % viability |
|---|---|---|
| | Density (540 nm) | |
| Untreated control | 0,531 | 100% |
| Vaseline treated control | 0,515 | 97% |
| 2% grape seed OPC palmitic ester | 0,703 | 132% |
| 5% undecylenated grape-seed OPC | 0,664 | 125% |
| 5% silylether grape seed OPC | 0,659 | 124% |

These results indicate that the tested products have no cytotoxicity compared to the untreated groups. This fact supports the use of the derivatized polyphenols obtained according to the process of the present invention in many applications, including, but not limited to, pharmaceutical applications, cosmetic applications, and food applications for human and animal nutrition. As the derivatized polyphenols obtained according to the process of the invention tend to have pronounced lipophilic characteristics, and no longer form complexes with proteins, their absorption through the gastrointestinal tract is facilitated. Once absorbed, they will be hydrolysed by the esterases and other enzymes present. The hydrolysed products, i.e. the polyphenols, fatty acids or silanols will thus be available to exert their influence and numerous advantageous properties.

The derivatized polyphenols obtained according to the process of the present invention can thus be used in any suitable form, such as tablets, gel capsules, creams, emulsions, face masks, lotions, washes, gels or even in solution. Excipients that can be associated therewith will be dependent on the formulation that it is desired to create. For example, for tablets, one would consider using starch, sodium laurylsulfate, magnesium stearate, lactose, microcrystalline cellulose, colloidal silica, sodium stearylfumarate, mannitol, gum arabic, talcum, and/or beeswax as appropriate. For gel capsules, the capsule coat excipients could be gelatine and silicone dioxides. As for creams, gels and lotions, the excipients would usually be those known for external application, for example colloidal silica, sodium hydroxide, demineralised water, carbopol, cetyl alcohol, stearyl alcohol, butylene glycol, glycerol stearate, glycerine, gum arabic, xanthan gum, isopropyl myristate., isononyl isononanoate, caprylic/capric triglyceride, cyclomethicone, dimethicone, etc.

The further following non-limitative examples of formulations are enclosed. The amounts indicated are weight/weight and the formulations are prepared according to usual cosmetic formulation practices.

### Anti-age Cream

| | |
|---|---|
| Glyceryl stearate | 2.50 |
| Octyldodecanol | 3.00 |
| Caprylic/ capric triglyceride | 3.0 |
| Cetearyl isononanoate | 2.0 |
| Dimethicone | 3.00 |
| Tribehenin PEG-20 esters | 3.0 |
| Grapeseed OPC ester of the invention | 0.7 |
| Argan oil | 3.0 |
| Squalane | 3.0 |
| Xanthan gum | 0.25 |
| Glycerine | 4.0 |
| Phenoxyethanol; caprylyl glycol; chlorphenesin | 0.7 |
| Perfume | 0.1 |
| Deionised water | QSP |

### Hair restructurant and protector formulation

| | |
|---|---|
| Cetearvl Alcohol & Ceteareth-20 | 10 |
| Octyldodecanol | 2 |
| Silvlether grape seed OPC according to the invention | 0.7 |
| Cocamide DEA | 1 |
| Osmosed water | QSP |
| Glycerine | 6 |
| Cetrimonium chloride | 0.5 |
| Phenoxyethanol, caprylyl glycol | 0.9 |
| Perfume | 0.2 |

### Anti-dandruff shampoo formulation

| | |
|---|---|
| Acrylates/ C10-30 Alkyl Acrvlate Crosspolymer | 1.2 |
| Propylene glycol | 6.0 |
| Sodium hydroxide | 0.4 |
| Sodium laureth sulfate | 30.0 |
| PEG-6 Caprylic/ capric glycerides | 3 |
| Undecvlenated grape seed OPC of the invention | 0.7 |
| Cocamidopropyl Betaine | 5.0 |
| DMDM hydantoine, iodopropylbutylcarbamate | 0.1 |
| Deionised water | QSP |

### Anti-oxidant and sun filter cream

| | |
|---|---|
| PEG-30 Dipolyhydroxystearate | 1.0 |
| Isohexadecane | 6.0 |
| Grape seed OPC ester of the invention | 0.5 |
| C-12-15 Alkylbenzoate, titanium oxide, polyhydroxystearic acid, aluinium stearate, alumina | 10.0 |
| Magnesium sulfate | 1.0 |
| Butylene glycol | 4.5 |
| Cvclopentasiloxane | 2.0 |
| Diazolidinvl Urea; Iodopropynyl Butylcarbamate | 0.6 |
| Deionised water | QSP |

## Claims

1. Process for the preparation of derivatized polyphenols comprising causing a polyphenol to react with an ester-forming or ether-forming functional group in the presence of an organic base, wherein :
- the polyphenol is chosen from:
- hydroxystilbenes selected from the group consisting of monomeric or oligomeric resveratrols, rhapontin, deoxyrhapontin, and piceatannol;
- hydroxycinnamic acids selected from the group consisting of rosmarinic acid, chlorogenic acids, caffeic acids, and ferulic acids;
- simple and analogous phenols selected from the group consisting of hydroxytyrosol, oleuropein, and verbascoside;
- flavan-3-ol monomers and oligomers selected from the group consisting of catechin, epicatechin, proanthocyanidine oligomers, gallocatechins, and epigallocatechins;
- flavonols, dihydroflavonols, flavanonols and isoflavones;
- hydroxychalcones and their derivatives, preferably aspalathin; and
- hydrolysable tannins selected from the group consisting of tannic acid, nobotannin, potentillin, and gallnut extract;
- the organic base is selected from the group consisting of imidazole derivatives soluble in water, ethanol and acetone wherein the organic base is chosen from the group consisting of 1-methylimidazole, 2-methylimidazole, 4-methylimidazole, 2-ethylimidazole, 1-ethylimidazole, 1-propylimidazole, and 1-isopropylimidazole; and
- the reaction is carried out in an aprotic solvent.

2. Process according to claim 1, wherein the aprotic solvent is chosen from the group consisting of acetone, ethyl acetate, isopropyl acetate, methylethylketone, and isopropyl ether.

3. Process according to any one of the preceding process claims, wherein the polyphenol is at least one of the following :
- a monomeric and/or oligomeric proanthocyanidine (OPC) found in the group consisting of green tea extract, grape-seed extract, pine bark extract, potentilla extract, and cocoa bean extract;
- a chalcone found in unfermented rooibois tea extract;
- a hydroxystilbene found in grape vine shoot extract.

4. Process according to any one of the preceding process claims, wherein the polyphenol is selected from the group consisting of : 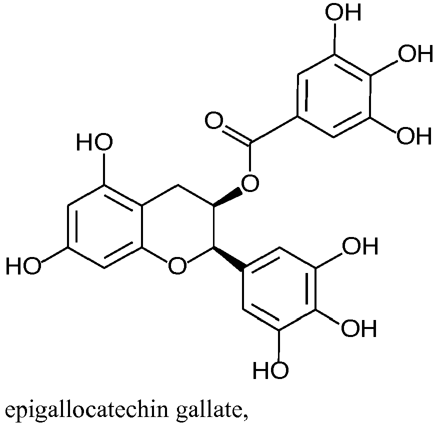 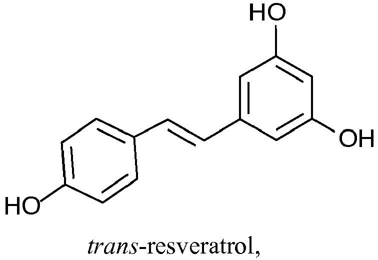 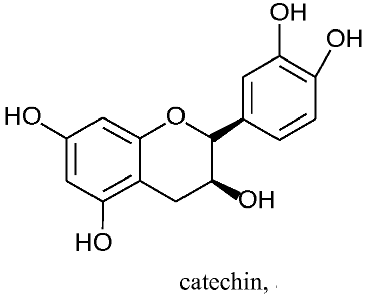 and

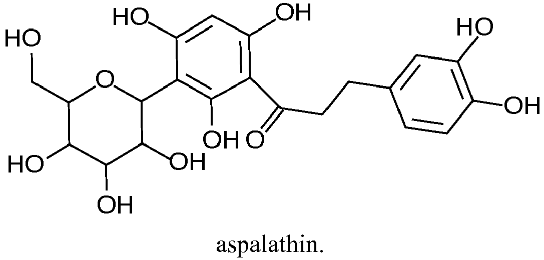

5. Process according to any one of the preceding process claims, wherein the ester-forming group originates from a fatty acid halide selected from the group consisting of saturated and unsaturated fatty acid halides containing 6-18 carbon atoms.

6. Process according to any one of the preceding claims, wherein the ether-forming group originates from silyl ethers as defined by formulae I and II hereunder : 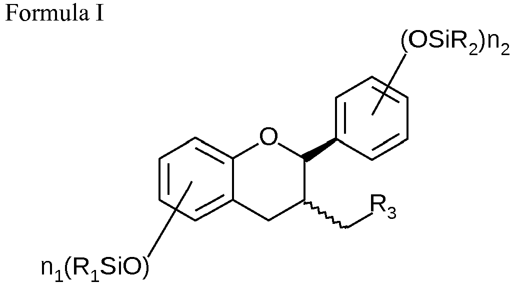 in which
- R₁ and R₂ are identical or different, linked to the Si atom by non-hydrolysable bonds, these radicals being saturated or unsaturated, substituted or unsubstituted hydrocarbons containing from between 1 to 30 carbon atoms, and when substituted contain one or more functional groups;
- R₃ is OH, H, or a silyl ether (OsiR), where R is identical or different to R₁ and R₂ as defined above.
n₁ and n₂ are identical or different, and have a value of from 0 to 3, corresponding to the number of substitutions on a ring; or 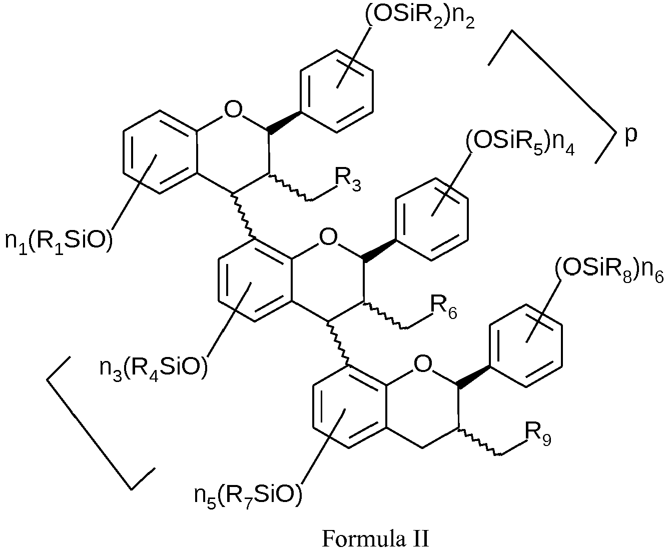 in which:
- n₁, n₂, n₃, n₄, n₅ and n₆ are identical or different, having a value of from 0 to 3 and corresponding to the number of substitutions on a ring;
- R₁, R₂, R₄, R₅, R₇, R₈ are identical or different, linked to the Si atom via non-hydrolysable bonds, and are saturated or unsaturated, substituted or unsubstituted, hydrocarbons containing, when substituted, several functional groups, the hydrocarbons containing from 1 to 30 carbon atoms ;
- R₃ , R₆, and R₉ are identical or different, preferably OH, H, or a silyl ether (OsiR), where R is identical or different to R₁ and R₂ as defined above;
- p is an integer from 0 to 10.

7. Process according to any one of the preceding process claims wherein the ether-forming group originates from tertbutyldimethyl chlorosilane.

## Patentansprüche

1. Herstellungsverfahren zum Herstellen von Polyphenolderivaten umfassend eine Reaktion eines Polyphenols mit einer Ester- oder Ether-bildenden funktionellen Gruppe in Gegenwart einer anorganischen Base, wobei :
- das Polyphenol aus folgenden Polyphenolen ausgewählt ist:
- Hydroxystilbenen, ausgewählt aus der Gruppe bestehend aus den Monomeren oder Oligomeren von Resveratrol, Rhapontin, Deoxyrhapontin, und Piceatannol ;
- Hydroxyzimtsäuren, ausgewählt aus der Gruppe bestehend aus Rosmarinsäure, Chlorogensäure, Kaffeinsäuren, und Ferulinsäuren ;
- Einfachen Phenolen und PhenolAnalogen, ausgewählt aus der Gruppe bestehend aus Hydroxytyrosol, Oleuropein, und Verbascosid ;
- Monomeren und Oligomeren von Flavan-3-ol, ausgewählt aus der Gruppe bestehend aus Catechin, Epicatechin, Oligomeren von Proanthocyanidinen, Gallocatechinen und den Epigallocatechinen ;
- Flavonolen, Dihydroflavonolen, Flavanonolen und Isoflavonen ;
- Hydroxychalkonen und Derivaten davon, bevorzugsweise Aspalathin ; und
- Hydrolysierbaren Tanninen, ausgewählt aus der Gruppe bestehend aus Tanninsäure, Nobotannin, Potentillin, und Gallapfelextrakt ;
- die organische Base aus der Gruppe bestehend aus in Wasser, Ethanol und Aceton löslich Imidazolderivaten ausgewählt ist, wobei die organische Base aus der Gruppe bestehend aus 1-Methylimidazol, 2-Methylimidazole 4-Methylimidazol, 2-Ethylimidazol, 1-Ethylimidazol, 1-Propylimidazol, und 1-Isopropylimidazol ausgewählt ist ; und
- die Reaktion in einem aprotischen Lösungsmittel durchgeführt wird.

2. Herstellungsverfahren nach Anspruch 1, wobei das aprotische Lösungsmittel aus der Gruppe bestehend aus Aceton, Ethlyacetat, Isopropylacetat, Methylethylketon, und Isopropylether ausgewählt ist.

3. Herstellungsverfahren nach irgendeinem der vorherigen Ansprüche, wobei das Polyphenol ein der folgenden Polyphenolen ist:
- ein sich in der Gruppe bestehend aus Gruntee-Extrakt, Traubenkernextrakt, Kiefernrindenextrakt, Potentillenextrakt, und Kakaobohnenextrakt befindendes Monomer und/oder Oligomer von Proanthocyanidin (OPC) ;
- ein sich in unfermentiertem Rooibostee-Extrakt befindendes Chalkon ;
- ein sich in Weinrebenextrakt befindendes Hydroxystilben.

4. Herstellungsverfahren nach irgendeinem der vorherigen Ansprüche, wobei das Polyphenol ausgewählt ist aus der Gruppe bestehend aus : 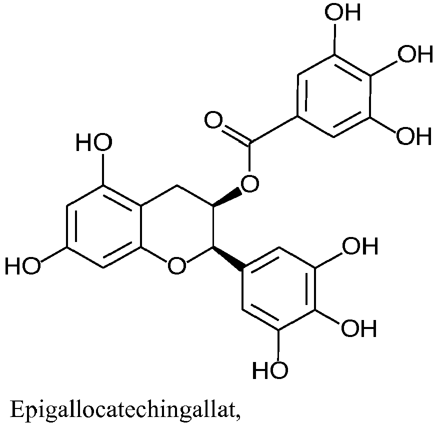 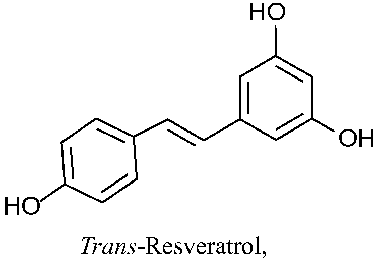 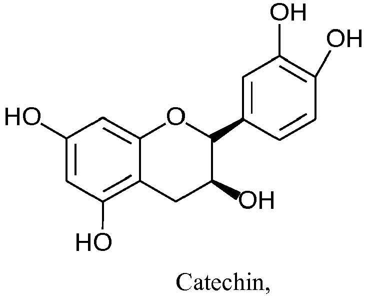 und

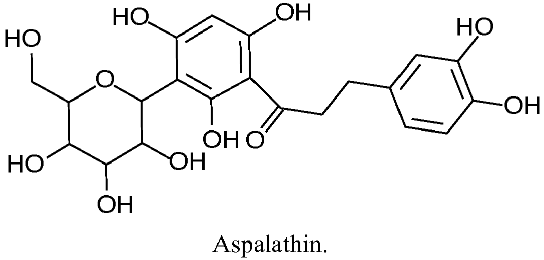

5. Herstellungsverfahren nach irgendeinem der vorherigen Ansprüche, wobei die Esterbildende Gruppe aus einem Fettsäurehalogenid stammt, ausgewählt aus der Gruppe bestehend aus den gesättigten und ungesättigten Fettsäurehalogeniden enthaltend 6 bis 18 Kohlenatome.

6. Herstellungsverfahren nach irgendeinem der vorherigen Ansprüche, wobei die Ether-bildende Gruppe aus Silyletheren stammt, die per unterstehenden Formeln I und II beschrieben sind : 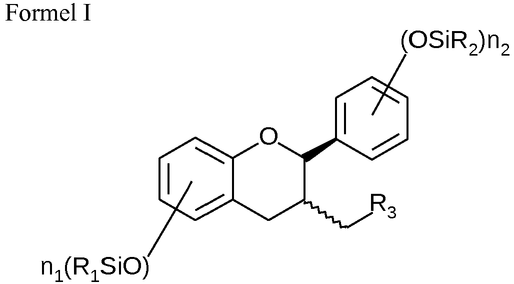 worin :
- R₁ und R₂, die gleich oder verschieden sind, mit dem Atom Si über nicht-hydrolysierbaren Verbindungen verbunden sind, diese Reste gesättigte oder ungesättigte, substituierte oder unsubstituierte, Kohlenwasserstoffreste sind, enthaltend 1 bis 30 Kohlenatomen, und wenn substituiert ein oder mehreren funktionellen Gruppen enthalten ;
- R₃ ist OH, H, oder ein Silylether (OSiR), wo R gleich oder verschieden ist zu bereits oben beschriebenen R₁ und R₂;
n₁ und n₂, die gleich oder verschieden sind, mit einem Wert von 0 bis 3, welches die Substituierungsnummer im Ring darstellt; oder 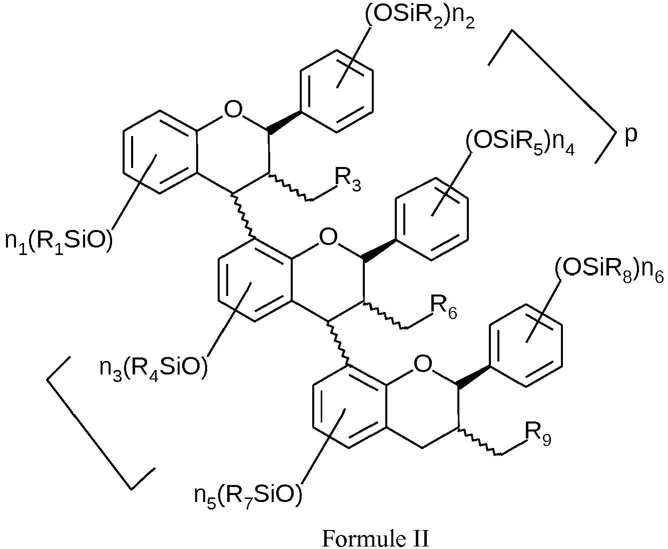 worin :
- n₁, n₂, n₃, n₄, n₅ und n₆, die gleich oder verschieden sind, mit einem Wert zwischen 0 und 3, welches die Substituierungsnummer im Ring darstellt;
- R₁, R₂, R₄, R₅, R₇, R₈, die gleich oder verschieden sind, und mit dem Atom Si über nicht-hydrolysierbaren Verbindungen verbunden sind, gesättigte oder ungesättigte, substituierte oder unsubstituierte, Kohlenwasserstoffreste sind, enthaltend, wenn substituiert, mehrere funktionellen Gruppe, die Kohlenwasserstoffreste enthaltend von 1 bis 30 Kohlenatome ;
- R₃ , R₆, und R₉, die gleich oder verschieden sind, bevorzugsweise OH, H, oder ein Silylether (OSiR) sind, wo R gleich oder verschieden sind zu oben beschriebenen R₁ und R₂ ;
- p ein Integer von 0 bis 10 ist.

7. Herstellungsverfahren nach einem der vorherigen Ansprüche, wobei die Ether-bildende Gruppe aus Tertbutyldimethylchlorosilane stammt.

## Revendications

1. Procédé d'obtention de dérivés de polyphénols comprenant le fait de faire réagir un polyphénol avec un groupe fonctionnel formant un ester ou un éther en présence d'une base organique, selon lequel :
- le polyphénol est choisi parmi :
- les hydroxystilbènes choisis dans le groupe consistant en les monomères ou oligomères de resvératrol, le rhapontin, le déoxyrhapontin, et le picéatannol ;
- les acides hydroxycinnamiques choisis dans le groupe consistant en l'acide rosmarinique, l'acide chlorogénique, les acides cafféiques, et les acides feruliques ;
- les phénols simples et analogues choisis dans le groupe consistant en l'hydroxytyrosol, l'oleuropéine, et le verbascoside ;
- les monomères et oligomères de flavan-3-ol choisis dans le groupe consistant en la catéchine, l'épicatéchine, les oligomères de proanthocyanidine, les gallocatéchines, et les épigallocatéchines ;
- les flavonols, les dihydroflavonols, les flavanonols et les isoflavones ;
- les hydroxychalcones et leurs dérivés, de préférence l'aspalathine ; et
- les tannins hydrolysables choisis dans le groupe consistant en l'acide tannique, la nobotanine, la potentilline,et l'extrait de noix de galle ;
- la base organique étant choisie dans le groupe consistant en des dérivés d'imidazole solubles dans l'eau, l'éthanol et l'acétone, selon lequel la base organique est choisie dans le groupe consistant en le 1-méthylimidazole, le 2-méthylimidazole, le 4-méthylimidazole, le 2-éthylimidazole, le 1-éthylimidazole, le 1-propylimidazole, et le 1-isopropylimidazole ; et
- la réaction est effectuée dans un solvant aprotique.

2. Procédé d'obtention selon la revendication 1, selon lequel le solvant aprotique est choisi dans le groupe consistant en l'acétone, l'acétate d'éthyle, l'acétate d'isopropyle, le méthyléthylcétone, et l'éther d'isopropyle.

3. Procédé d'obtention selon l'une quelconque des revendications précédentes, selon lequel le polyphénol est au moins l'un des polyphénols suivants :
- un monomère et/ou oligomère de proanthocyanidine (OPC) se trouvant dans le groupe consistant en l'extrait de thé vert, l'extrait de pépins de raisin, l'extrait d'écorce de pin, l'extrait de potentille, et l'extrait de fève de cacao ;
- une chalcone se trouvant dans l'extrait de thé rooibos non-fermenté ;
- un hydroxystilbène se trouvant dans l'extrait de pousse de vigne à vin.

4. Procédé d'obtention selon l'une quelconque des revendications précédentes, selon lequel le polyphénol est choisi dans le groupe consistant en : 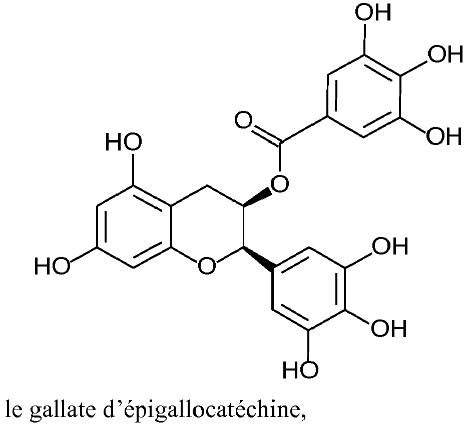 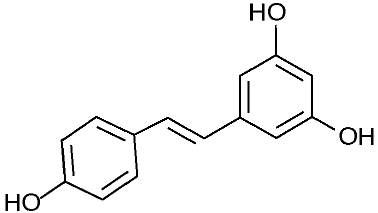 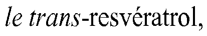 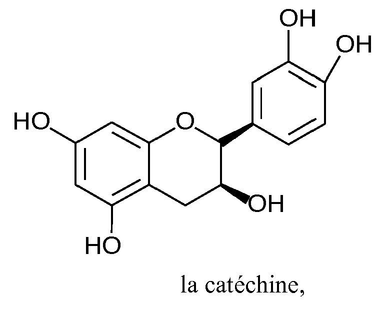 et

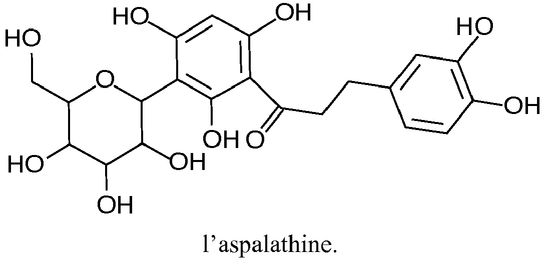

5. Procédé d'obtention selon l'une quelconque des revendications précédentes, selon lequel le groupe formant un ester provient d'un halogénure d'acide gras choisi dans le groupe consistant en les halogénures d'acide gras saturé et non-saturé contenant de 6 à 18 atomes de carbone.

6. Procédé d'obtention selon l'une quelconque des revendications précédentes, selon lequel le groupe formant un éther provient d'éthers de silyle tels que définis par les formules I et II ci-après : 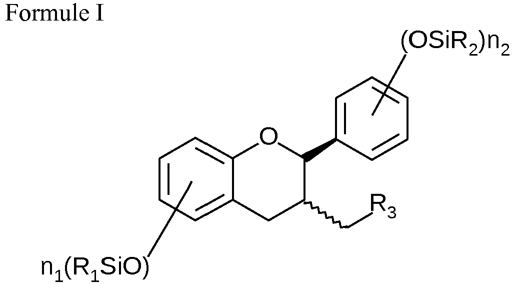 dans laquelle :
- R₁ et R₂, identiques ou différents, sont liés à l'atome Si par des liaisons non-hydrolysables, ces radicaux étant des hydrocarbures saturés ou non-saturés, substitués ou non-substitués contenant entre 1 à 30 atomes de carbone, et lorsque substitués contiennent un ou plusieurs groupes fonctionnels ;
- R₃ est OH, H, ou un éther desilyle (OSiR), où R est identique à, ou différent de, R₁ et R₂ tels que définis ci-dessus ;
n₁ et n₂, identiques ou différents, d'une valeur entre 0 et 3, correspondant au nombre de substitutions sur un cycle ; ou 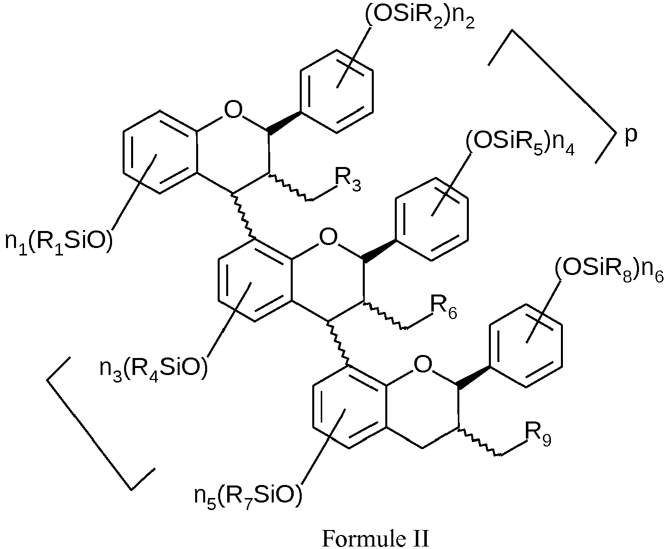 dans laquelle :
- n₁, n₂, n₃, n₄, n₅ et n₆, identiques ou différents, d'une valeur entre 0 et 3, correspondant au nombre de substitutions sur un cycle ;
- R₁, R₂, R₄, R₅, R₇ , R₈, identiques ou différents, liés à l'atome Si via des liaisons non-hydrolysables, sont des hydrocarbures saturés ou non-saturés, substitués ou non-substitués, contenant, lorsque substitués, plusieurs groupes fonctionnels, les hydrocarbures contenant de 1 à 30 atomes de carbone ;
- R₃ , R₆, et R₉, identiques ou différents, de préférence OH, H, ou un éther de silyle (OSiR), où R est identique à, ou différent de, R₁ et R₂ tels que définis ci-dessus ;
- p est un entier de 0 à 10.

7. Procédé d'obtention selon l'une quelconque des revendications précédentes, selon lequel le groupe formant un éther provient du tertbutyldiméthylchlorosilane.
